# EUROPEAN PATENT APPLICATION

(11) **EP 3 315 078 A1**
(43) Date of publication of application: **02.05.2018**
(21) Application number: 16814401.2
(22) Date of filing: 22.06.2016
(51) Int. Cl.: A61B 8/14, A61B 8/12

(54) **ULTRASONIC VIBRATOR AND ULTRASONIC PROBE**

(30) Priority: 23.06.2015 JP 2015126051
(71) Applicant: Olympus Corporation, Hachioji-shi Tokyo 192-8507 (JP)
(72) Inventor: YOSHIDA, Satoshi, Hachioji-shi Tokyo 192-8507 (JP)
(74) Representative: Gunzelmann, Rainer
(86) International application number: PCT/JP2016/068538
(87) International publication number: WO 2016/208631

(57) **Abstract**

An ultrasound transducer according to the present invention includes a plurality of ultrasound elements, each of the ultrasound elements including at least an element configured to emit an ultrasound wave according to an input of an electrical signal and convert the ultrasound wave entered from an outside into an echo signal, and a plurality of acoustic matching layers laminated on the element and configured to match acoustic impedance between the element and an observation target, and a connecting portion protruding to an opposite side of the element with respect to a plane passing through a surface of the acoustic matching layer, and configured to connect the ultrasound elements adjacent to each other among the plurality of ultrasound elements.

## Description

### Field

The present invention relates to an ultrasound transducer that emits ultrasound waves to an observation target, and receives an ultrasound echo reflected at an observation target, converts the ultrasound echo into an echo signal, and outputs the echo signal, and an ultrasound probe.

### Background

Ultrasound waves are sometimes applied to observe characteristics of living tissues or materials that are objects to be observed. To be specific, an ultrasound transducer transmits an ultrasound wave to an observation target and receives an ultrasound echo reflected at the observation target, and an ultrasound observation apparatus applies predetermined signal processing to the received ultrasound echo to obtain an image of the observation target and information on its characteristics.

The ultrasound transducer includes an element that converts an electrical pulse signal into an ultrasound pulse (acoustic pulse) and irradiates the observation target with the ultrasound pulse, and converts the ultrasound echo reflected at the observation target into an electrical echo signal expressed by voltage change and outputs the echo signal, and a plurality of elements (ultrasound elements) laminated on the element, each of the elements including at least an acoustic matching layer that matches acoustic impedance between the element and the observation target. For example, the plurality of ultrasound elements is arranged along a predetermined direction, and the ultrasound element involved in transmission and reception is electronically switched and the transmission and reception of the ultrasound elements are delayed, whereby the ultrasound echo is acquired from the observation target.

As a method of manufacturing such an ultrasound transducer, a technology of bonding a base material made of a piezoelectric material to a sheet made of a material that configures a backing material, and dividing the base material by dicing to form a plurality of piezoelectric elements is disclosed (refer to Patent Literature 1, for example). In Patent Literature 1, the sheet on which the plurality of piezoelectric elements is formed is bent along an array direction of the piezoelectric elements and is bonded to the backing material having a curved surface, whereby a convex-type ultrasound transducer is manufactured.

### Citation List

### Patent Literature

Patent Literature 1: JP 4-26418 A

### Summary

### Technical Problem

By the way, to improve directivity characteristics (element factors) of the ultrasound elements, it is favorable that the ultrasound wave emission side between the adjacent ultrasound elements is completely divided. In Patent Literature 1, the ultrasound elements are diced from the ultrasound wave emission side such that adjacent ultrasound elements are completely divided. However, in the case of bending the sheet on which the plurality of ultrasound elements is arrayed, a pitch on a side different from the side bonded with the sheet, that is, on the ultrasound wave emission side, in the adjacent ultrasound element, is larger than a pitch of when the ultrasound elements are divided by dicing.

The present invention has been made in view of the foregoing, and an objective is to provide an ultrasound transducer and an ultrasound probe that improve a directional characteristic of ultrasound elements, and can maintain a pitch of the ultrasound elements on a side emitting ultrasound waves when the ultrasound elements are bent along an array direction to a pitch at the time of division.

### Solution to Problem

To solve the above-described problem and achieve the object, an ultrasound transducer according to the present invention includes: a plurality of ultrasound elements, each of the ultrasound elements including at least an element configured to emit an ultrasound wave according to an input of an electrical signal and convert the ultrasound wave entered from an outside into an echo signal, and one or a plurality of acoustic matching layers laminated on the element and configured to match acoustic impedance between the element and an observation target; and a connecting portion protruding to an opposite side of the element with respect to a plane passing through a surface of the acoustic matching layer, and configured to connect the ultrasound elements adjacent to each other among the plurality of ultrasound elements.

Moreover, in the above-described ultrasound transducer according to the present invention, the plurality of ultrasound elements form a shape bent along an array direction.

Moreover, in the above-described ultrasound transducer according to the present invention, the element is provided with a ground electrode for grounding, and the connecting portion is formed using a conductive material, and is electrically connected with the ground electrode.

Moreover, in the above-described ultrasound transducer according to the present invention, the connecting portion is formed using a same material as the acoustic matching layer.

Moreover, in the above-described ultrasound transducer according to the present invention, the ultrasound element has a plurality of acoustic matching layers, and the connecting portion is formed using a same material as the acoustic matching layer to be connected.

Moreover, the above-described ultrasound transducer according to the present invention further includes: a backing material configured to attenuate ultrasound vibration caused by an operation of the element, wherein the connecting portion is formed using a same material as the backing material.

Moreover, the above-described ultrasound transducer according to the present invention further includes: an acoustic lens configured to emit the ultrasound wave passed through the acoustic matching layer to an outside, wherein the connecting portion is formed using a same material as the acoustic lens.

Moreover, an ultrasound probe according to the present invention includes the ultrasound transducer according to the above-described invention at a distal end.

Moreover, the above-described ultrasound probe according to the present invention further includes an ultrasound endoscope including the ultrasound transducer at a distal end, and an insertion portion to be inserted into a subject.

### Advantageous Effects of Invention

According to the present invention, effect to improve a directional characteristic of ultrasound elements, and maintain a pitch of the ultrasound elements on a side emitting ultrasound waves when the ultrasound elements are bent along an array direction to a pitch at the time of division is exhibited.

### Brief Description of Drawings

FIG. 1 is a diagram schematically illustrating an endoscope system according to a first embodiment of the present invention.
FIG. 2 is a perspective view schematically illustrating a distal end configuration of an insertion portion of an ultrasound endoscope according to the first embodiment of the present invention.
FIG. 3 is a perspective view schematically illustrating a configuration of an ultrasound transducer according to the first embodiment of the present invention.
FIG. 4 is a plan view schematically illustrating the configuration of the ultrasound transducer viewed from an arrow A direction illustrated in FIG. 3.
FIG. 5 is a plan view schematically illustrating the configuration of the ultrasound transducer viewed from an arrow B direction illustrated in FIG. 3.
FIG. 6 is a schematic diagram illustrating a method of manufacturing the ultrasound transducer according to the first embodiment of the present invention.
FIG. 7 is a schematic diagram illustrating the method of manufacturing the ultrasound transducer according to the first embodiment of the present invention.
FIG. 8 is a schematic diagram illustrating the method of manufacturing the ultrasound transducer according to the first embodiment of the present invention.
FIG. 9 is a schematic diagram illustrating the method of manufacturing the ultrasound transducer according to the first embodiment of the present invention.
FIG. 10 is a plan view schematically illustrating a configuration of an ultrasound transducer according to a modification of the first embodiment of the present invention.
FIG. 11 is a perspective view schematically illustrating a configuration of an ultrasound transducer according to a second embodiment of the present invention.
FIG. 12 is a plan view schematically illustrating a configuration of the ultrasound transducer viewed from an arrow C direction illustrated in FIG. 11.
FIG. 13 is a plan view schematically illustrating the configuration of the ultrasound transducer viewed from an arrow D direction illustrated in FIG. 11.
FIG. 14 is a schematic diagram illustrating a method of manufacturing the ultrasound transducer according to the second embodiment of the present invention.
FIG. 15 is a schematic diagram illustrating the method of manufacturing the ultrasound transducer according to the second embodiment of the present invention.
FIG. 16 is a schematic diagram illustrating the method of manufacturing the ultrasound transducer according to the second embodiment of the present invention.
FIG. 17 is a schematic diagram illustrating a method of manufacturing an ultrasound transducer according to a third embodiment of the present invention.
FIG. 18 is a schematic diagram illustrating the method of manufacturing an ultrasound transducer according to the third embodiment of the present invention.
FIG. 19 is a schematic diagram illustrating a method of manufacturing an ultrasound transducer according to a first modification of the third embodiment of the present invention.
FIG. 20 is a schematic diagram illustrating the method of manufacturing an ultrasound transducer according to the first modification of the third embodiment of the present invention.
FIG. 21 is a top view schematically illustrating a principal portion of an ultrasound transducer according to a second modification of the third embodiment of the present invention.
FIG. 22 is a top view schematically illustrating a principal portion of an ultrasound transducer according to a third modification of the third embodiment of the present invention.
FIG. 23 is a top view schematically illustrating a principal portion of an ultrasound transducer according to a fourth modification of the third embodiment of the present invention.
FIG. 24 is a top view schematically illustrating a principal portion of an ultrasound transducer according to a fifth modification of the third embodiment of the present invention.

### Description of Embodiments

Hereinafter, modes for carrying out the present invention (hereinafter, embodiments) will be described with reference to the drawings. Note that the present invention is not limited by the embodiments described below. Further, the same portion is denoted with the same sign in the illustration of the drawings.

### (First Embodiment)

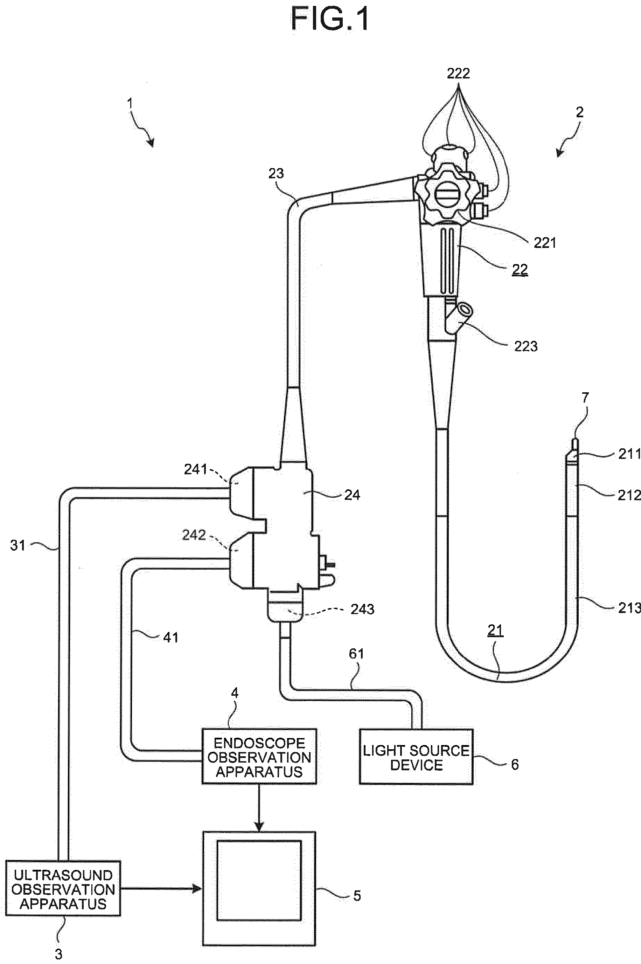

FIG. 1 is a diagram schematically illustrating an endoscope system according to a first embodiment of the present invention. An endoscope system 1 is a system that performs ultrasound diagnosis of an inside of a subject such as a human being, using an ultrasound endoscope. As illustrated in FIG. 1, the endoscope system 1 includes an ultrasound endoscope 2, an ultrasound observation apparatus 3, an endoscope observation apparatus 4, a display device 5, and a light source device 6.

The ultrasound endoscope 2 converts, at a distal end portion thereof, an electrical pulse signal received from the ultrasound observation apparatus 3 into an ultrasound pulse (acoustic pulse) and irradiates a subject with the ultrasound pulse, and converts an ultrasound echo reflected at the subject into an electrical echo signal expressed by voltage change and outputs the echo signal.

The ultrasound endoscope 2 typically includes an imaging optical system and an imaging element, and can be inserted into digestive tract (esophagus, stomach, duodenum, large intestine) or respiratory tract (trachea/bronchus) of the subject and can capture the digestive tract and the respiratory tract. Further, the ultrasound endoscope 2 can capture surrounding organs (pancreas, gallbladder, bile duct, biliary tract, lymph node, mediastinum, blood vessels, and the like), using ultrasound waves. Further, the ultrasound endoscope 2 includes a light guide that guides illumination light to be radiated to the subject at the time of optical capturing. The light guide has a distal end portion reaching a distal end of an insertion portion to the subject, of the ultrasound endoscope 2, and a proximal end portion connected to the light source device 6 that generates illumination light.

As illustrated in FIG. 1, the ultrasound endoscope 2 includes an insertion portion 21, an operating unit 22, a universal cable 23, and a connector 24. The insertion portion 21 is a portion to be inserted into the subject. As illustrated in FIG. 1, the insertion portion 21 includes an ultrasound transducer 7 provided on a distal end side, a rigid member 211 that holds the ultrasound transducer 7, a bendable bend portion 212 connected to a proximal end side of the rigid member 211, and a flexible tube portion 213 connected to a proximal end side of the bend portion 212 and having flexibility. Here, although specific illustration is omitted, a light guide that transmits the illumination light supplied from the light source device 6, and a treatment instrument insertion passage in which a plurality of signal cables that transmits various signals is routed and which allows a treatment instrument to be inserted therethrough are formed inside the insertion portion 21.

The ultrasound transducer 7 may be any one of a convex transducer, a linear transducer, and a radial transducer. In the first embodiment, the ultrasound endoscope 2 has a plurality of piezoelectric elements provided in an array manner as the ultrasound transducer 7, and electronically switches the piezoelectric element involved in transmission and reception and delays the transmission and reception of the piezoelectric elements, to cause the ultrasound transducer 7 to electronically perform scanning. However, the ultrasound transducer 7 may mechanically perform scanning. The configuration of the ultrasound transducer 7 will be described below.

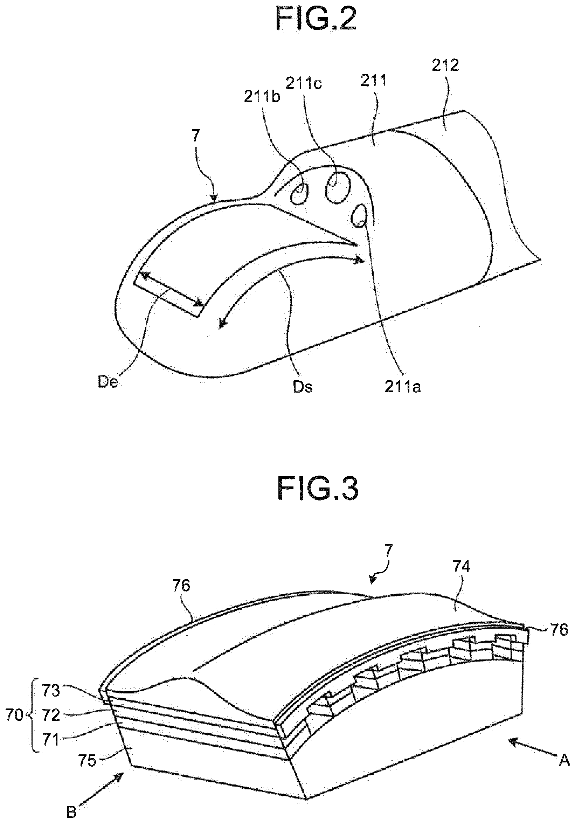

FIG. 2 is a perspective view schematically illustrating a distal end configuration of the insertion portion of the ultrasound endoscope according to the first embodiment. As illustrated in FIG. 2, the rigid member 211 includes an illumination lens 211a that collects the illumination light and emits the light to an outside, an objective lens 211b that forms a part of the imaging optical system and takes in light from an outside, and a treatment instrument protruding port 211c communicating with the treatment instrument insertion passage formed in the insertion portion 21, and allows the treatment instrument to protrude from the distal end of the insertion portion 21.

The operating unit 22 is a portion connected to a proximal end side of the insertion portion 21 and receiving various operations from a doctor or the like. As illustrated in FIG. 1, the operating unit 22 includes a bending knob 221 for bending and operating the bend portion 212 and a plurality of operating members 222 for performing various operations. Further, a treatment instrument insertion port 223 communicating with the treatment instrument insertion passage and for allowing the treatment instrument to be inserted into the treatment instrument insertion passage is formed in the operating unit 22.

The universal cable 23 is a cable extending from the operating unit 22 and in which a plurality of signal cables that transmit various signals and an optical fiber that transmits the illumination light supplied from the light source device 6 are disposed.

The connector 24 is provided at a distal end of the universal cable 23. The connector 24 includes first to third connector portions 241 to 243 to which an ultrasound cable 31, a video cable 41, and an optical fiber cable 61 are respectively connected.

The ultrasound observation apparatus 3 is electrically connected to the ultrasound endoscope 2 via the ultrasound cable 31 (see FIG. 1), and outputs a pulse signal to the ultrasound endoscope 2 via the ultrasound cable 31 and inputs an echo signal from the ultrasound endoscope 2. Then, the ultrasound observation apparatus 3 applies predetermined processing to the echo signal to generate an ultrasound image.

The endoscope observation apparatus 4 is electrically connected to the ultrasound endoscope 2 via the video cable 41 (see FIG. 1), and inputs an image signal from the ultrasound endoscope 2 via the video cable 41. Then, the endoscope observation apparatus 4 applies predetermined processing to the image signal to generate an endoscope image.

The display device 5 is configured from liquid crystal or organic electro luminescence (EL), a projector, a cathode ray tube (CRT), and the like, and displays the ultrasound image generated by the ultrasound observation apparatus 3, the endoscope image generated by the endoscope observation apparatus 4, and the like.

The light source device 6 is connected to the ultrasound endoscope 2 via the optical fiber cable 61 (see FIG. 1), and supplies, to the ultrasound endoscope 2, the illumination light for illuminating the inside of the subject via the optical fiber cable 61.

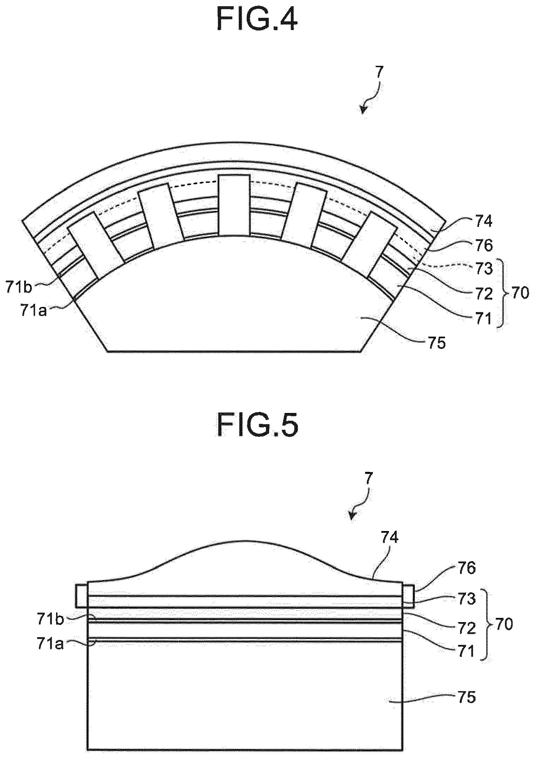

Next, a configuration of the ultrasound transducer 7 provided at the distal end of the insertion portion 21 will be described with reference to FIGS. 2 to 6. FIG. 3 is a perspective view schematically illustrating a configuration of the ultrasound transducer according to the first embodiment. FIG. 4 is a plan view schematically illustrating the configuration of the ultrasound transducer viewed from an arrow A direction illustrated in FIG. 3. FIG. 5 is a plan view schematically illustrating the configuration of the ultrasound transducer viewed from an arrow B direction illustrated in FIG. 3. Note that FIGS. 3 and 4 illustrate the ultrasound transducer 7 in which six piezoelectric elements 71 are arranged. However, FIGS. 3 and 4 are diagrams of a simplified configuration of the ultrasound transducer 7 for description and the actually disposed number is not limited to six. In the first embodiment, the ultrasound transducer 7 is a convex ultrasound transducer as illustrated in FIG. 2 and is a one-dimensional array (1D array) in which the plurality of piezoelectric elements 71 is arrayed in a line. In other words, in the ultrasound transducer 7 according to the first embodiment, the plurality of piezoelectric elements 71 is arranged along an outer surface that forms a curved surface of the ultrasound transducer 7.

The ultrasound transducer 7 includes the plurality of piezoelectric elements 71 having a prismatic shape and aligned in a longitudinal direction, a plurality of first acoustic matching layers 72 respectively provided on the piezoelectric elements 71 on the outer surface side of the ultrasound transducer 7, a plurality of second acoustic matching layers 73 provided on the first acoustic matching layers 72 on an opposite side of a side being in contact with the piezoelectric elements 71, an acoustic lens 74 provided on the second acoustic matching layers 73 on an opposite side of a side being in contact with the first acoustic matching layers 72, a backing material 75 provided on the piezoelectric elements 71 on an opposite side of a side being in contact with the first acoustic matching layers 72, and a connecting portion 76 that connects adjacent ultrasound elements 70. Note that the first embodiment has a configuration in which the first acoustic matching layer 72 and the second acoustic matching layer 73 are provided for each piezoelectric element 71, and the acoustic lens 74 and the backing material 75 collectively cover the plurality of piezoelectric elements 71, first acoustic matching layers 72, and second acoustic matching layers 73. The ultrasound element 70 according to the first embodiment includes the piezoelectric element 71, the first acoustic matching layer 72, and the second acoustic matching layer 73. The ultrasound transducer 7 may use one piezoelectric element 71 as an output unit or a plurality of piezoelectric elements 71 as an output unit. Hereinafter, as illustrated in FIG. 2, the longitudinal direction of the piezoelectric element 71 is referred to as an elevation direction De, and the array direction of the piezoelectric elements 71 is referred to as a scanning direction Ds.

The piezoelectric element 71 converts an electrical pulse signal into an acoustic pulse and irradiates a subject with the acoustic pulse, and converts an ultrasound echo reflected at the subject into an electrical echo signal expressed by voltage change and outputs the echo signal. The piezoelectric element 71 is provided with a signal input/output electrode 71a on a principal plane on the backing material 75 side, and a ground electrode 71b for grounding on a principal plane of the piezoelectric element 71 on the first acoustic matching layer 72 side. The electrodes are formed using a conductive metal material or resin material.

The piezoelectric element 71 is formed using lead zirconate titanate (PZT) ceramic material, PMN-PT single crystal, PMN-PZT single crystal, PZN-PT single crystal, PIN-PZN-PT single crystal, or relaxor material. The PMN-PT single crystal is an abbreviation of a solid solution of lead magnesium niobate and lead titanate. The PMN-PZT single crystal is an abbreviation of a solid solution of lead magnesium niobate and lead zirconate titanate. The PZN-PT single crystal is an abbreviation of a solid solution of lead zinc niobate and lead titanate. The PIN-PZN-PT single crystal is an abbreviation of a solid solution of lead indium niobate, lead zinc niobate, and lead titanate. The relaxor material is a generic term for a three-component piezoelectric material obtained by adding lead-based complex perovskite that is a relaxor material to PZT for the purpose of increasing a piezoelectric constant and a dielectric constant. Lead-based complex perovskite is represented by Pb(B1, B2)O₃. B1 is any one of magnesium, zinc, indium, or scandium, and B2 is any one of niobium, tantalum, or tungsten. These materials have an excellent piezoelectric effect. Therefore, the value of the electrical impedance can be lowered even if the device is downsized, which is favorable from the viewpoint of impedance matching with a thin-film electrode provided in the piezoelectric element 71.

To efficiently transmit sound (ultrasound wave) between the piezoelectric element 71 and the observation target, the first acoustic matching layer 72 and the second acoustic matching layer 73 match acoustic impedance between the piezoelectric element 71 and the observation target. The first acoustic matching layer 72 and the second acoustic matching layer 73 are made of different materials from each other. Note that, in the first embodiment, description is given on the assumption that the two acoustic matching layers (the first acoustic matching layer 72 and the second acoustic matching layer 73) are provided. However, one layer or three or more layers may be employed depending on the characteristics of the piezoelectric element 71 and the observation target.

The acoustic lens 74 is formed using silicone, polymethylpentene, an epoxy resin, polyetherimide, or the like, and one plane is formed into a convex shape or a concave shape and has a function to narrow down ultrasound waves. The acoustic lens 74 emits the ultrasound wave having passed through the second acoustic matching layer 73 to an outside or takes in the ultrasound echo from an outside. The acoustic lens 74 may be arbitrarily provided and may not be provided.

The backing material 75 attenuates unnecessary ultrasound vibration caused by an operation of the piezoelectric element 71. The backing material 75 is formed using a material having a large attenuation factor, such as an epoxy resin in which a filler of alumina or zirconia is dispersed, or a rubber in which the above-described filler is dispersed.

The connecting portion 76 is formed using the same material as the second acoustic matching layer 73. The connecting portion 76 has a comb shape extending along the scanning direction Ds, and a notch corresponding to an interval of the second acoustic matching layer 73 is formed. The connecting portion 76 is bonded to both ends of the second acoustic matching layer 73 in the elevation direction De, thereby to connect the adjacent second acoustic matching layers 73 to connect the ultrasound elements 70. The connecting portion 76 may be bonded to the piezoelectric element 71 and the first acoustic matching layer 72. The connecting portion 76 is bonded to at least the second acoustic matching layer 73 located on an outer peripheral side when the ultrasound elements 70 are bent as a convex type and connects the adjacent second acoustic matching layers 73. The connecting portion 76 protrudes from a curved surface that passes through a surface of the second acoustic matching layer 73 on an opposite side of a side facing the first acoustic matching layer 72. Note that the connecting portion 76 may be integrally provided with the second acoustic matching layer 73. Further, the connecting portion 76 is favorably arranged in an area out of an area (effective area) where the piezoelectric element 71 transmits and receives the ultrasound wave.

The ultrasound transducer 7 having the above configuration irradiates the observation target with an ultrasound wave via the first acoustic matching layer 72, the second acoustic matching layer 73, and the acoustic lens 74 as the piezoelectric element 71 is vibrated by an input of the pulse signal. At this time, in the piezoelectric element 71, the vibration of the piezoelectric element 71 is attenuated and is not transmitted by the backing material 75 on the opposite side of the side where the first acoustic matching layer 72, the second acoustic matching layer 73, and the acoustic lens 74 are disposed. Further, the ultrasound wave reflected from the observation target is transmitted to the piezoelectric element 71 via the first acoustic matching layer 72, the second acoustic matching layer 73, and the acoustic lens 74. The piezoelectric element 71 is vibrated by the transmitted ultrasound wave, and the piezoelectric element 71 converts the vibration into an electrical echo signal and outputs the echo signal to the ultrasound observation apparatus 3 as an echo signal via wiring (not illustrated).

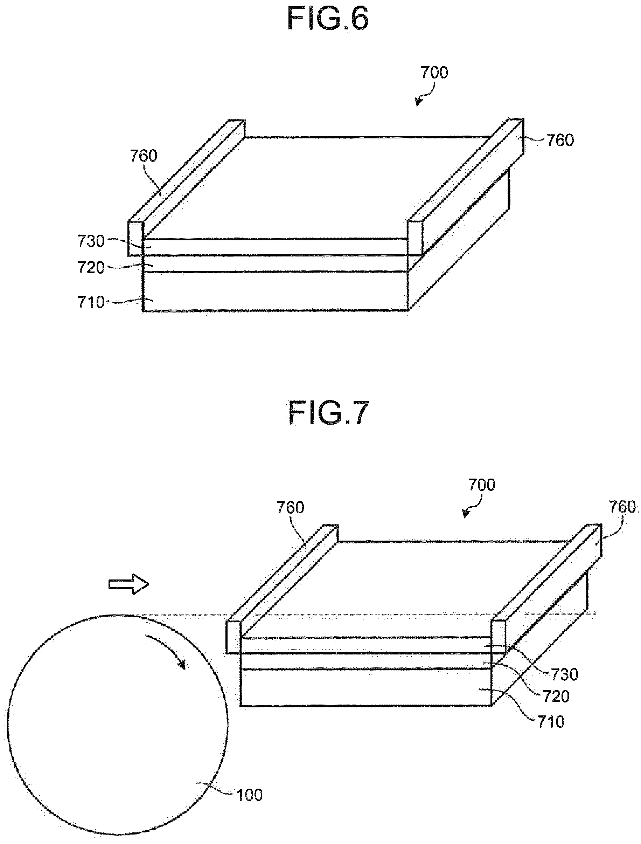

Next, a method of manufacturing the above-described ultrasound transducer 7 will be described with reference to FIGS. 6 to 9. FIGS. 6 to 9 are schematic diagrams illustrating the method of manufacturing the ultrasound transducer according to the first embodiment of the present invention. In the method of manufacturing the ultrasound transducer 7, first, a forming member 700 for forming the piezoelectric element 71, the first acoustic matching layer 72, and the second acoustic matching layer 73 is manufactured.

To be specific, a rectangular parallelopiped first acoustic matching layer base material 720 formed using a material that configures the first acoustic matching layer 72 is laminated on one facing principal plane of a rectangular parallelopiped piezoelectric element base material 710 made of a piezoelectric material, and a rectangular parallelopiped second acoustic matching layer base material 730 formed using a material that configures the second acoustic matching layer 73 is laminated on a principal plane that is a surface of the first acoustic matching layer base material 720 and on an opposite side of a side of the piezoelectric element base material 710. The base materials are bonded with, for example, an adhesive through which ultrasound waves can pass. Note that the description will be given on the assumption that the material that configures the signal input/output electrode 71a and the ground electrode 71b is laminated on the piezoelectric element base material 710. After that, a prismatic-shaped connecting portion base material 760 formed using a material that configures the connecting portion 76 is bonded to facing two end portions of the second acoustic matching layer base material 730 in the laminated base material, and to end portions forming side surfaces orthogonal to a lamination surface. The connecting portion base material 760 protrudes from a plane that passes through a surface of the second acoustic matching layer base material 730 and a surface on an opposite side of a contact side of the first acoustic matching layer base material 720. In this way, the forming member 700 in which the connecting portion base material 760 is bonded to the laminated base material having the piezoelectric element base material 710, the first acoustic matching layer base material 720, and the second acoustic matching layer base material 730 laminated in this order is manufactured (see FIG. 6). Note that a base material in which the second acoustic matching layer base material 730 and the connecting portion base material 760 are integrally formed may be used.

Next, to form a plurality of the ultrasound elements 70 including the piezoelectric element 71, the first acoustic matching layer 72, and the second acoustic matching layer 73, division of the forming member 700, for example, division by dicing is performed. For example, a blade 100 that is a dicing blade as illustrated in FIG. 7 is used, and the forming member 700 is cut by moving the blade 100 along a dividing direction while rotating the blade 100. At this time, the forming member 700 is not divided and is cut in such a manner that a part of the forming member 700 is connected. Here, the dividing direction in the first embodiment refers to a direction orthogonal to the principal planes (lamination surface) of the piezoelectric element base material 710, the first acoustic matching layer base material 720, and the second acoustic matching layer base material 730, and to the longitudinal direction of the connecting portion base material 760. Further, the blade 100 is moved while maintaining a position where an outer edge of the blade 100 slightly protrudes from the surface of the second acoustic matching layer base material 730 and the blade 100 does not disconnect the connecting portion base material 760.

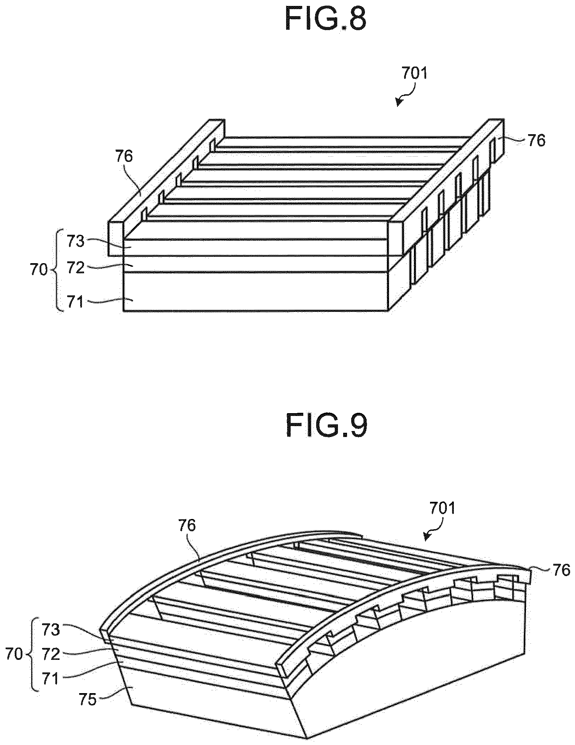

By the cutting of the forming member 700 with the blade 100, a laminate 701 in which the piezoelectric element base material 710, the first acoustic matching layer base material 720, and the second acoustic matching layer base material 730 are divided, and the connecting portion base material 760 forms a comb shape is obtained (see FIG. 8). Through this process, a plurality of ultrasound elements 70 including the piezoelectric element 71, the first acoustic matching layer 72, and the second acoustic matching layer 73, and the connecting portion 76 are formed.

After that, the laminate 701 obtained by cutting is bent, the piezoelectric element 71 side is joined to the backing material 75 (see FIG. 9), and the acoustic lens 74 is provided on the second acoustic matching layer 73 side, whereby the ultrasound transducer 7 illustrated in FIG. 3 can be obtained. In the first embodiment, the plurality of second acoustic matching layers 73 is connected by the connecting portion 76, and thus a pitch between the second acoustic matching layers 73 becomes nearly equal to a pitch divided by dicing even if the laminate 701 is bent. Therefore, a pitch of the first acoustic matching layer 72 bonded to the second acoustic matching layer 73 and a pitch of the piezoelectric element 71 bonded to the first acoustic matching layer 72 become nearly equal to or narrower than the pitch divided by dicing, and refinement becomes possible. That is, in the first embodiment, the second acoustic matching layers 73 are connected by the connecting portion 76 to maintain the pitch between the second acoustic matching layers 73, and thus the pitch on an outer peripheral side when the laminate 701 is bent becomes nearly equal to or narrower than the pitch on an inner peripheral side that is the piezoelectric element 71 side, and refinement becomes possible. Note that "nearly equal" referred here includes a design error, change in pitch due to elongation of the connecting portion 76, and the like.

Note that, in the above-described manufacturing method, the example in which the plurality of piezoelectric elements 71 and the like are formed by dicing the forming member 700 with the blade 100 has been described. However, the formed body illustrated in FIG. 8 may be manufactured by formation using laser, formation by etching, or formation using a mold. Further, in the above-described manufacturing method, the example in which the acoustic lens 74 is provided after the bent laminate 701 is attached to the backing material 75 has been described. However, the laminate 701 may be attached to the backing material 75 after the acoustic lens 74 is provided on the bent laminate 701.

In the first embodiment described above, in the forming member 700 obtained by bonding the connecting portion base material 760 to the laminated base material having a substantially prismatic shape in which the piezoelectric element base material 710, the first acoustic matching layer base material 720, and the second acoustic matching layer base material 730 are laminated in this order, the piezoelectric element base material 710, the first acoustic matching layer base material 720, and the second acoustic matching layer base material 730 are divided by dicing or the like to manufacture the laminate 701 in which the plurality of piezoelectric elements 71, the plurality of first acoustic matching layers 72, the plurality of second acoustic matching layers 73, and the connecting portion 76 are formed, and the acoustic lens 74 and the backing material 75 are attached to the laminate 701 bent in a state where the adjacent ultrasound elements 70 are maintained at the pitch at the time of division by the connecting portion 76, whereby the ultrasound transducer 7 is manufactured. As a result, the directional characteristic of the ultrasound element 70 is improved and the pitch of the ultrasound elements 70 on the side emitting the ultrasound waves when the plurality of ultrasound elements 70 is bent along the array direction can be maintained to the pitch at the time of division.

### (Modification of First Embodiment)

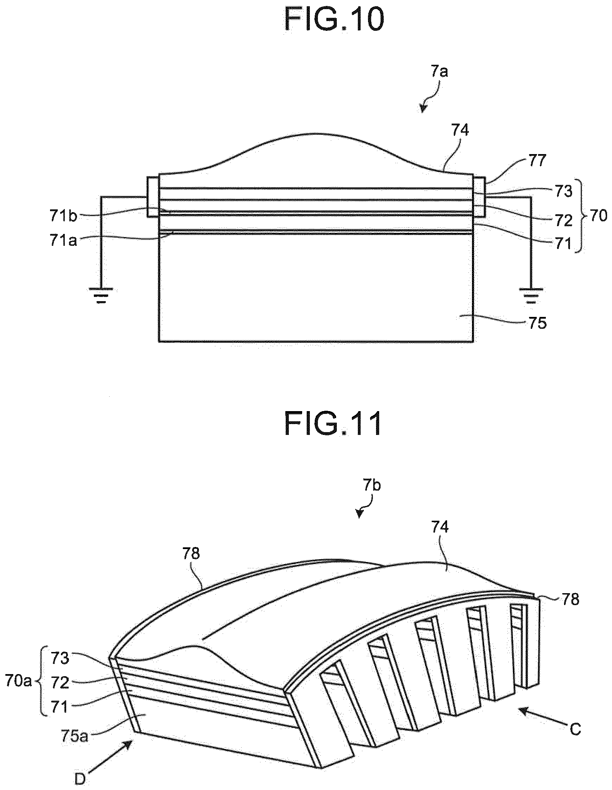

FIG. 10 is a plan view schematically illustrating a configuration of an ultrasound transducer according to a modification of the first embodiment of the present invention. FIG. 10 is a plan view corresponding to the arrow B direction illustrated in FIG. 3. In the above-described first embodiment, the example in which the connecting portion 76 is formed using the same material as the second acoustic matching layer 73 has been described. However, in the present modification, a conductive material is used as a material of a connecting portion. An ultrasound transducer 7a according to the present modification includes a connecting portion 77 in place of the connecting portion 76 of the ultrasound transducer 7 described above.

The connecting portion 77 is formed using a conductive material mixed with a conductive paste such as a silver paste. The connecting portion 77 is formed by, similarly to the manufacturing method according to the above-described first embodiment, bonding a prismatic-shaped connecting portion base material formed using a material that configures the connecting portion 77 to two facing end portions of a laminated base material, and to end portions forming side surfaces orthogonal to a lamination surface, and dicing the laminated base material. In the present modification, one end of the connecting portion 77 protrudes from an end surface of a second acoustic matching layer 73, and the other end extends to a ground electrode 71b and is connected to the ground electrode 71b, in a laminating direction of a piezoelectric element 71, a first acoustic matching layer 72, and the like. The connecting portion 77 and the ground electrode 71b are bonded with a conductive adhesive or the like. In addition, the connecting portion 77 is grounded to a ground potential, and each of the piezoelectric elements 71 is grounded to the ground potential via the connecting portion 77.

According to the present modification, the connecting portion 77 formed using the conductive material is grounded to the ground potential. Therefore, a pitch at the time of bending ultrasound elements 70 can be nearly equal to a pitch at the time of division, and the connecting portion 77 can function as a ground connecting electrode. Further, in the present modification, the piezoelectric elements 71 and the like can be formed and the connection electrode for grounding the piezoelectric elements 71 to the ground potential can be formed only by division by dicing or the like.

Further, in the first embodiment and the modification described above, the examples in which the connecting portions 76 and 77 are provided to both end portions in the elevation direction De have been described. However, the connecting portions 76 and 77 may be provided to only one of the both end portions. The connecting portions 76 and 77 may only support the plurality of second acoustic matching layers 73 at least one end side.

Further, in the first embodiment and the modification described above, the examples in which the connecting portion 76 or 77 is formed using the second acoustic matching layer 73 or the conductive material has been described. However, the connecting portions 76 and 77 may be formed using the same material as the acoustic lens 74 or the same material as the first acoustic matching layer 72, or may be formed using a bendable material capable of maintaining the pitch at the time of division, for example, a metal material, unlike the first acoustic matching layer 72, the second acoustic matching layer 73, and the acoustic lens 74.

### (Second Embodiment)

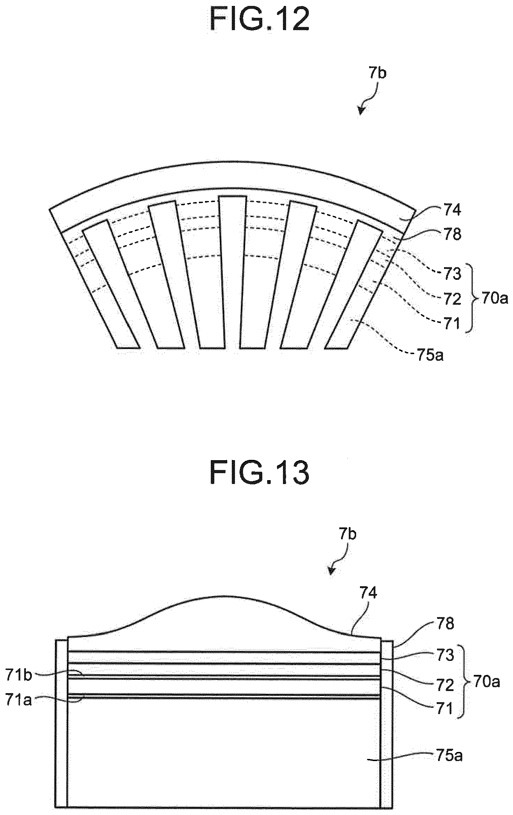

FIG. 11 is a perspective view schematically illustrating a configuration of an ultrasound transducer according to a second embodiment of the present invention. FIG. 12 is a plan view schematically illustrating a configuration of the ultrasound transducer viewed from an arrow C direction illustrated in FIG. 11. FIG. 13 is a plan view schematically illustrating the configuration of the ultrasound transducer viewed from an arrow D direction illustrated in FIG. 11. In the above-described first embodiment, the example in which the connecting portion 76 is formed using the same material as the second acoustic matching layer 73 has been described. However, in the second embodiment, a connecting portion is formed using the same material as a backing material 75a.

An ultrasound transducer 7b according to the second embodiment includes a plurality of piezoelectric elements 71, a plurality of first acoustic matching layers 72, a plurality of second acoustic matching layers 73, an acoustic lens 74, a plurality of backing materials 75a provided on the piezoelectric elements 71 on an opposite side of a side being in contact with the first acoustic matching layers 72, and a connecting portion 78 that connects adjacent ultrasound elements 70a. The ultrasound element 70a according to the second embodiment includes the piezoelectric element 71, the first acoustic matching layer 72, and the second acoustic matching layer 73.

The connecting portion 78 is formed using the same material as the backing material 75a. The connecting portion 78 forms a comb shape and covers end portions on an elevation direction De side, of the piezoelectric elements 71, the first acoustic matching layers 72, the second acoustic matching layers 73, and the backing materials 75a. The connecting portion 78 is bonded to both ends of the second acoustic matching layer 73 in the elevation direction De, thereby to connect the adjacent second acoustic matching layers 73. The connecting portion 78 protrudes from a surface of the second acoustic matching layer 73 on an opposite side of a side of the first acoustic matching layer 72. The connecting portion 78 may just be a connecting portion bonded to at least one of the piezoelectric element 71, the first acoustic matching layer 72, and the second acoustic matching layer 73, and connecting the adjacent ultrasound elements 70a via the backing materials 75a when at least the ultrasound elements 70a are bent as a convex type. Note that the connecting portion 78 may be integrally provided with the backing material 75a.

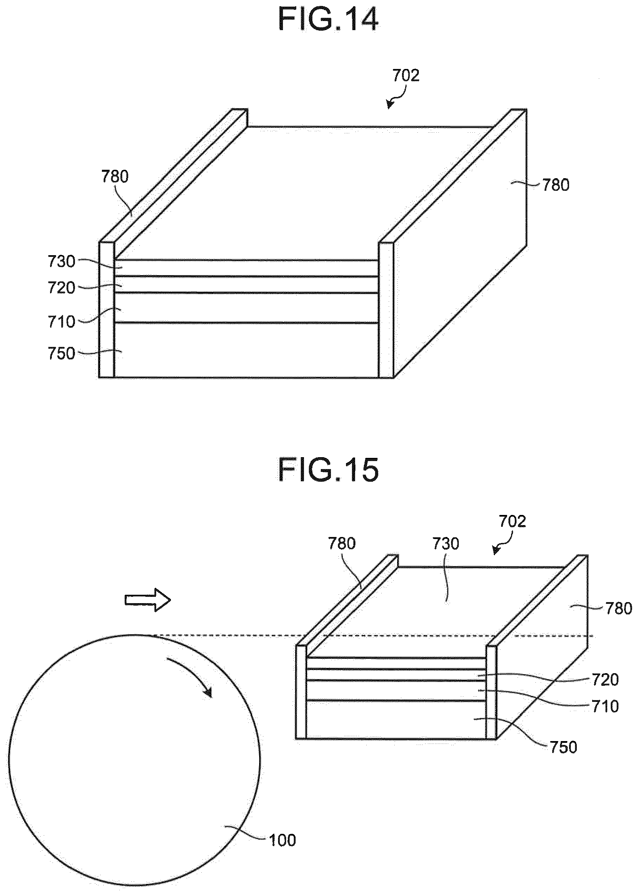

Next, a method of manufacturing the above-described ultrasound transducer 7b will be described with reference to FIGS. 14 to 16. FIGS. 14 to 16 are schematic diagrams illustrating a method of manufacturing the ultrasound transducer according to the second embodiment of the present invention. In the method of manufacturing the ultrasound transducer 7b, first, a forming member 702 for forming the piezoelectric element 71, the first acoustic matching layer 72, the second acoustic matching layer 73, and the backing material 75a is manufactured.

To be specific, as described above, a piezoelectric element base material 710, a first acoustic matching layer base material 720, and a second acoustic matching layer base material 730 are laminated, and a rectangular parallelopiped backing base material 750 formed using a material that configures the backing material 75a is laminated on the other facing principal plane of the piezoelectric element base material 710, to manufacture a laminated base material. After that, a planar connecting portion base material 780 formed using a material that configures the connecting portion 78 is bonded to facing two side surfaces of the laminated base material, and to side surfaces exposing the piezoelectric element base material 710, the first acoustic matching layer base material 720, the second acoustic matching layer base material 730, and the backing base material 750 in the lamination order. The connecting portion base material 780 is disposed to cover the piezoelectric element base material 710, the first acoustic matching layer base material 720, the second acoustic matching layer base material 730, and the backing base material 750. In this way, the forming member 702 in which the connecting portion base material 780 is bonded to the laminated base material having the backing base material 750, the piezoelectric element base material 710, the first acoustic matching layer base material 720, and the second acoustic matching layer base material 730 laminated in this order is manufactured (see FIG. 14).

Next, to form a plurality of the ultrasound elements 70a including the piezoelectric element 71, the first acoustic matching layer 72, and the second acoustic matching layer 73, division of the forming member 702, for example, division by dicing is performed. For example, a blade 100 that is a dicing blade as illustrated in FIG. 15 is used, and the forming member 702 is cut by moving the blade 100 along a dividing direction while rotating the blade 100. At this time, the connecting portion base material 780 is not divided and is cut in such a manner that a part of the connecting portion base material 780 is connected. Here, the dividing direction in the second embodiment refers to a direction orthogonal to the principal planes (lamination surface) of the piezoelectric element base material 710, the first acoustic matching layer base material 720, the second acoustic matching layer base material 730, and the backing base material 750, and to the principal plane of the connecting portion base material 780. Further, the blade 100 is moved while maintaining a position where an outer edge of the blade 100 slightly protrudes from the surface of the second acoustic matching layer base material 730 and the blade 100 does not disconnect the connecting portion base material 780.

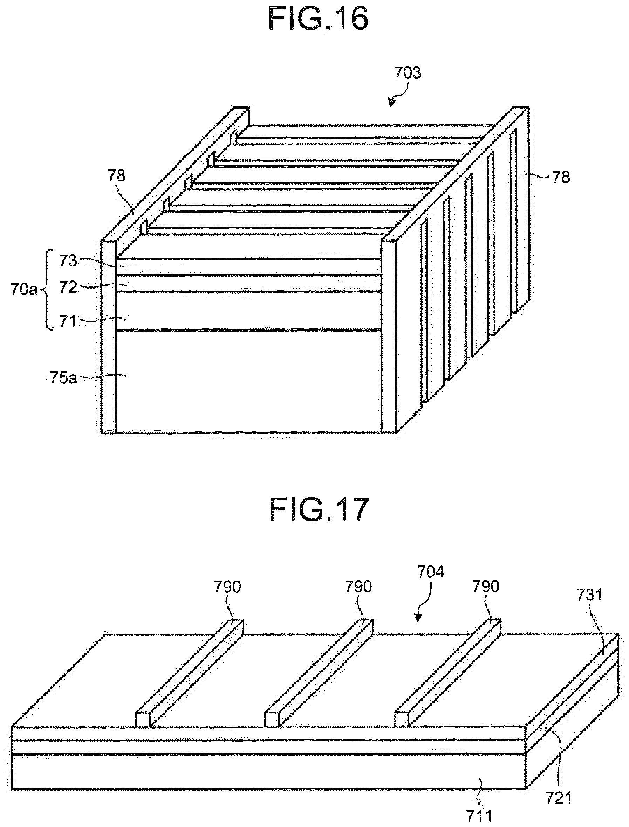

By the cutting of the forming member 702 with the blade 100, a laminate 703 in which the piezoelectric element base material 710, the first acoustic matching layer base material 720, the second acoustic matching layer base material 730, and the backing base material 750 are divided, and the connecting portion base material 780 forms a comb shape is obtained (see FIG. 16). Through this process, the plurality of ultrasound elements 70a including the piezoelectric element 71, the first acoustic matching layer 72, and the second acoustic matching layer 73, the plurality of backing materials 75a, and the connecting portion 78 are formed.

After that, the laminate 703 obtained by cutting is bent, and an acoustic lens 74 is provided on the second acoustic matching layer 73 side, whereby the ultrasound transducer 7b illustrated in FIG. 11 can be obtained. In the second embodiment, at least the plurality of second acoustic matching layers 73 is connected by the connecting portion 78, and thus a pitch between the ultrasound elements 70a becomes nearly equal to or narrower than a pitch at the time of division by dicing and refinement becomes possible, even if the laminate 703 is bent. That is, in the second embodiment, the second acoustic matching layers 73 are connected by the connecting portion 78 to maintain the pitch between the second acoustic matching layers 73, and thus the pitch on an outer peripheral side when the laminate 703 is bent becomes nearly equal to or narrower than the pitch on an inner peripheral side that is the piezoelectric element 71 side, and refinement becomes possible. Note that, in the ultrasound transducer 7b illustrated in FIGS. 11 and 12, the example in which a plane passing through the end surfaces of the backing material 75a forms a flat plane by applying surface treatment to the end portions of the backing materials 75a on an opposite side of the piezoelectric elements 71 has been described. However, the plane passing through the end surfaces of the backing materials 75a may form a curved surface without the surface treatment, or surface treatment may be applied to form a shape suitable for attachment to a distal end of an insertion portion 21.

In the second embodiment described above, in the forming member 702 obtained by bonding the connecting portion base material 780 to the laminated base material having a substantially prismatic shape in which the backing base material 750, the piezoelectric element base material 710, the first acoustic matching layer base material 720, and the second acoustic matching layer base material 730 are laminated in this order, the piezoelectric element base material 710, the first acoustic matching layer base material 720, the second acoustic matching layer base material 730, and the backing base material 780 are divided by dicing or the like to manufacture the laminate 703 in which the ultrasound element 70a formed of the plurality of piezoelectric elements 71, the plurality of first acoustic matching layers 72, and the plurality of second acoustic matching layers 73, the plurality of backing materials 75a, and the connecting portion 78 are formed, and the acoustic lens 74 is attached to the laminate 703 bent in a state where the adjacent second acoustic matching layers 73 are maintained at the pitch at the time of division by the connecting portion 78, whereby the ultrasound transducer 7 is manufactured. As a result, the directional characteristic of the ultrasound element 70a is improved and the pitch of the ultrasound elements 70a on the side emitting ultrasound waves when the ultrasound elements 70a are bent along an array direction can be maintained to the pitch at the time of division.

In the above-described second embodiment, the connecting portion 78 has been bonded to at least the second acoustic matching layer 73. However, in a case where the connecting portion 78 is integrally provided with the backing material 75a, a base material in which the backing base material 750 and the connecting portion base material 780 are integrated is used to manufacture the ultrasound transducer 7b. As a result, the manufacturing process and the number of parts of the manufactured ultrasound transducer 7b can be reduced, and the ultrasound transducer 7b can be easily manufactured.

### (Third Embodiment)

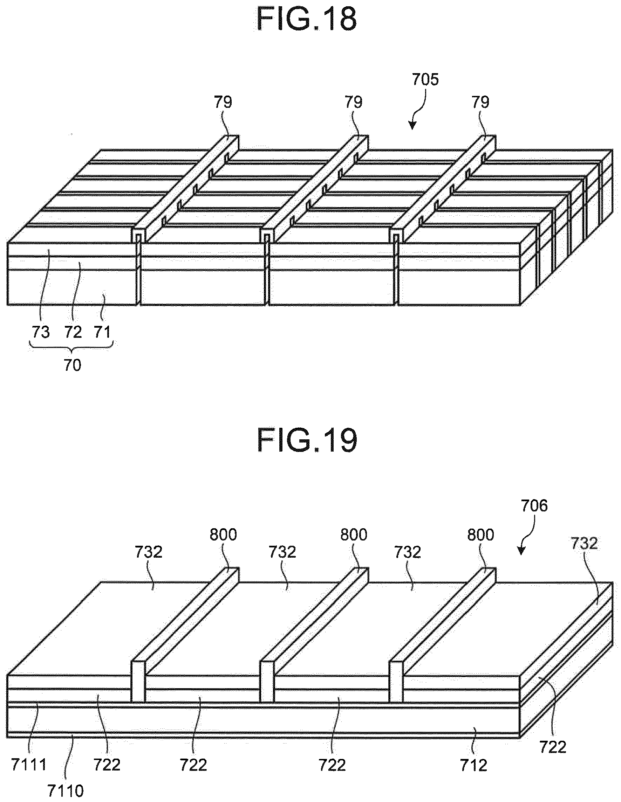

In the above-described first and second embodiments, the examples of manufacturing the 1D-array ultrasound transducer have been described. However, in a third embodiment, a method of manufacturing a 2D-array ultrasound transducer will be described. As the ultrasound transducer according to the present third embodiment, an example in which the plurality of piezoelectric elements 71, the plurality of first acoustic matching layers 72, and the plurality of second acoustic matching layers 73 described above are provided in a matrix manner, a backing material 75 is provided to a side of the piezoelectric elements 71 on an opposite side of a side being in contact with the first acoustic matching layers 72, and the adjacent second acoustic matching layers 73 are connected by a connecting portion 79 will be described (for example, see FIG. 18). In the present third embodiment, an ultrasound element is configured from the piezoelectric element 71, the first acoustic matching layer 72, and the second acoustic matching layer 73.

FIGS. 17 and 18 are schematic diagrams illustrating a method of manufacturing an ultrasound transducer according to the third embodiment of the present invention. In the method of manufacturing an ultrasound transducer according to the third embodiment, first, a forming member 704 for forming the piezoelectric element 71, the first acoustic matching layer 72, and the second acoustic matching layer 73 is manufactured.

To be specific, a rectangular parallelopiped first acoustic matching layer base material 721 formed using a material that configures the first acoustic matching layer 72 is laminated on one facing principal plane of a rectangular parallelopiped piezoelectric element base material 711 formed using a material that configures the piezoelectric element 71 (including a signal input/output electrode 71a and a ground electrode 71b), and a rectangular parallelopiped second acoustic matching layer base material 731 formed using a material that configures the second acoustic matching layer 73 is laminated on a principal plane that is a surface of the first acoustic matching layer base material 721 and on an opposite side of a side of the piezoelectric element base material 711 to manufacture the laminated base. After that, a plurality of connecting portion base materials 790 forming a prismatic shape and formed using a material that configures the connecting portion 79 is bonded to a principal plane of the laminated base material on the second acoustic matching layer base material 731 side. The plurality of connecting portion base materials 790 is arranged to be parallel to one another. In this way, the forming member 704 in which the connecting portion base material 790 is bonded to the laminated base material having the piezoelectric element base material 711, the first acoustic matching layer base material 721, and the second acoustic matching layer base material 731 laminated in this order is manufactured (see FIG. 17). Note that the connecting portion base material 790 is formed using a material that configures the first acoustic matching layer 72, the second acoustic matching layer 73, an acoustic lens, or the backing material.

Next, to form the piezoelectric element 71, the first acoustic matching layer 72, and the second acoustic matching layer 73, the forming member 704 is diced. For example, a blade 100 that is a dicing blade as illustrated in FIG. 7 is used, and the forming member 704 is cut by moving the blade 100 along a dividing direction while rotating the blade 100. At this time, the connecting portion base material 790 is not divided and is cut in such a manner that a part of the connecting portion base material 790 is connected. Here, the dividing direction in the third embodiment is two directions orthogonal to each other to divide a principal surface (lamination surface) of the forming member 704 in a matrix manner according to the arrangement of the piezoelectric elements 71. Further, the blade 100 is moved while maintaining a position where an outer edge of the blade 100 slightly protrudes from a surface of the second acoustic matching layer base material 731 and the blade 100 does not disconnect the connecting portion base material 790.

By the cutting of the forming member 704 with the blade 100, a laminate 705 in which the piezoelectric element base material 711, the first acoustic matching layer base material 721, and the second acoustic matching layer base material 731 are divided, one side surface of the connecting portion base material 790 forms a comb shape, and a side surface orthogonal to the side surface has a groove formed in a longitudinal direction is obtained (see FIG. 18). Through this process, the plurality of piezoelectric elements 71, the first acoustic matching layers 72, and the second acoustic matching layers 73, the plurality of backing materials arranged in a matrix manner, and the connecting portions 79 are formed.

After that, the laminate 705 obtained by cutting is bent, an acoustic lens is provided on the second acoustic matching layer 73 side, and the backing material is provided on the piezoelectric element 71 side, whereby the 2D-array ultrasound transducer can be obtained. In the third embodiment, the plurality of second acoustic matching layers 73 is connected by the connecting portion 79, and thus a pitch between the second acoustic matching layers 73 becomes nearly equal to or narrower than a pitch at the time of division by dicing and refinement becomes possible, even if the laminate 705 is bent.

In the third embodiment described above, in the forming member 704 obtained by bonding the connecting portion base material 790 to the laminated base material having a substantially prismatic shape in which the piezoelectric element base material 711, the first acoustic matching layer base material 721, and the second acoustic matching layer base material 731 are laminated in this order, the piezoelectric element base material 711, the first acoustic matching layer base material 721, and the second acoustic matching layer base material 731 are divided by dicing or the like to manufacture the laminate 705 in which the ultrasound elements including the plurality of piezoelectric elements 71, the plurality of first acoustic matching layers 72, the plurality of second acoustic matching layers 73, and the connecting portion 79 are formed, and the acoustic lens and the backing material are attached to the laminate 705 bent in a state where the adjacent second acoustic matching layers 73 are maintained at the pitch at the time of division by the connecting portion 79, whereby the ultrasound transducer 7 is manufactured. As a result, the directional characteristic of the ultrasound element is improved and the pitch of the ultrasound elements on the side emitting ultrasound waves when the ultrasound elements are bent along an array direction can be maintained to the pitch at the time of division.

### (First Modification of Third Embodiment)

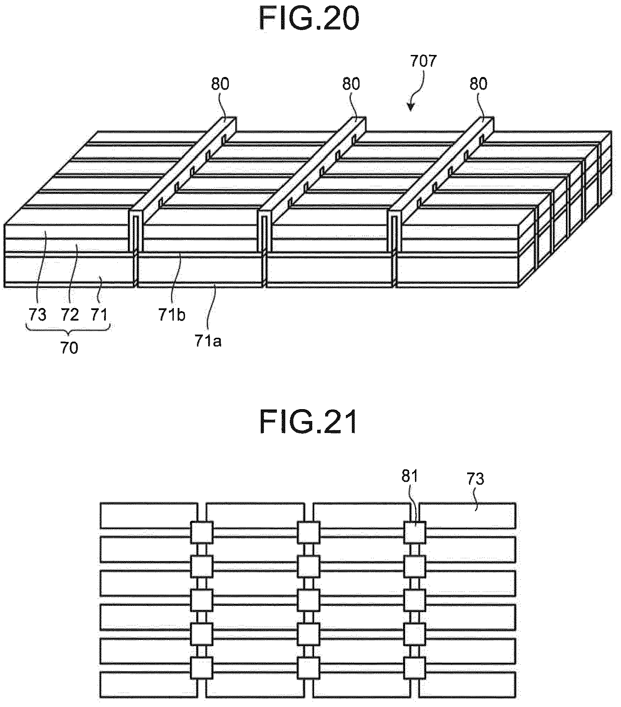

In the above-described third embodiment, the example in which the connecting portion is formed using the material that configures the first acoustic matching layer, the second acoustic matching layer, the acoustic lens, or the backing material has been described. However, in the present first modification, an example in which a connecting portion is formed using a conductive material will be described. As an ultrasound transducer according to the present first modification, an example in which the plurality of piezoelectric elements 71, the plurality of first acoustic matching layers 72, and the plurality of second acoustic matching layers 73 described above are provided in a matrix manner, an acoustic lens is provided on the plurality of second acoustic matching layers 73, a backing material is provided to a side of the piezoelectric elements 71 on an opposite side of a side being in contact with the first acoustic matching layers 72, and ground electrodes 71b of the adjacent piezoelectric elements 71 are connected by a connecting portion 80 will be described (for example, see FIG. 20). The connecting portion 80 is formed using a conductive material mixed with a conductive paste such as a silver paste, or a metal material.

FIGS. 19 and 20 are schematic diagrams illustrating a method of manufacturing the ultrasound transducer according to the first modification of the third embodiment of the present invention. In the method of manufacturing the ultrasound transducer according to the first modification, first, a forming member 706 for forming the piezoelectric element 71, the first acoustic matching layer 72, and the second acoustic matching layer 73 is manufactured.

To be specific, in a rectangular parallelopiped piezoelectric element base material 712 formed using a material that configures the piezoelectric element 71, provided with an electrode thin film 7110 formed using a material that configures a signal input/output electrode 71a on one principal plane, and provided with an electrode thin film 7111 formed using a material that configures the ground electrode 71b on the other principal plane, a plurality of connecting portion base materials 800 having a prismatic shape formed using a material that configures the connecting portion 80 is bonded to a principal plane of the electrode thin film 7111. The plurality of connecting portion base materials 800 is arranged to be parallel to one another. The electrode thin film 7111 and the connecting portion base material 800 are bonded with a conductive adhesive or the like.

After that, a plurality of first acoustic matching layer base materials 722 forming a rectangular parallelopiped shape and formed using a material that configures the first acoustic matching layer 72 is provided between the connecting portion base materials 800. After that, a plurality of second acoustic matching layer base materials 732 forming a rectangular parallelopiped shape and formed using a material that configures the second acoustic matching layer 73 is laminated on a surface of the first acoustic matching layer base material 722 and on a principal plane on an opposite side of the piezoelectric element base material 712 side to manufacture a laminate 707. In this way, the forming member 706 in which the piezoelectric element base material 712, the first acoustic matching layer base material 722, and the second acoustic matching layer base material 732 are laminated in this order, and the connecting portion base material 800 is provided between the first acoustic matching layer base materials 722 and between the second acoustic matching layer base materials 732 is manufactured (see FIG. 19).

Next, to form the piezoelectric element 71, the first acoustic matching layer 72, and the second acoustic matching layer 73, the forming member 706 is diced. For example, a blade 100 that is a dicing blade as illustrated in FIG. 7 is used, and the forming member 706 is cut by moving the blade 100 along a dividing direction while rotating the blade 100. At this time, the connecting portion base material 800 is not divided and is cut in such a manner that a part of the connecting portion base material 800 is connected.

By the cutting of the forming member 706 with the blade 100, a laminate 707 in which the piezoelectric element base material 712, the first acoustic matching layer base material 722, and the second acoustic matching layer base material 732 are divided, one side surface of the connecting portion base material 800 forms a comb shape, and a side surface orthogonal to the side surface has a groove along a longitudinal direction is obtained (see FIG. 20). Through this process, the plurality of piezoelectric elements 71, the first acoustic matching layers 72, and second acoustic matching layers 73, the backing materials arranged in a matrix manner and the connecting portion 80 are formed. Here, the connecting portion 80 connects the ground electrodes 71b of the adjacent piezoelectric elements 71 to each other.

After that, the laminate 707 obtained by cutting is bent, an acoustic lens is provided on the second acoustic matching layer 73 side, and the backing material is provided on the piezoelectric element 71 side, whereby the 2D-array ultrasound transducer can be obtained. In the ultrasound transducer according to the first modification, the connecting portion 80 is grounded to a ground potential, whereby to ground the piezoelectric elements 71 to the ground potential via the connecting portion 80.

According to the first modification, the connecting portion 80 formed using the conductive material is grounded to the ground potential. Therefore, a pitch at the time of bending the piezoelectric elements 71, the first acoustic matching layers 72, and the second acoustic matching layers 73 can be nearly equal to a pitch at the time of division, and the connecting portion 80 can function as a ground connecting electrode. Further, in the present modification, the piezoelectric elements 71 and the like can be formed and the connection electrode for grounding the piezoelectric elements 71 to the ground potential can be formed only by division by dicing or the like.

Note that, in the above-described first modification, the example in which the connecting portion 80 is directly bonded to the ground electrode 71b has been described. However, in the configuration of the third embodiment, the connecting portion 79 may be formed using a conductive material, a through-hole or the like may be formed in the first acoustic matching layer 72 and the second acoustic matching layer 73, and the ground electrode 71b and the connecting portion 79 may be electrically connected via the through-hole.

### (Second Modification of Third Embodiment)

FIG. 21 is a top view schematically illustrating a principal portion of an ultrasound transducer according to a second modification of the third embodiment of the present invention. In the above-described third embodiment, the example in which the connecting portion 79 collectively holds the second acoustic matching layers 73 according to the piezoelectric elements 71 arrayed in one direction of array directions of the piezoelectric elements 71, has been described. However, a plurality of connecting portions that holds only the second acoustic matching layers 73 to be connected may be provided. In the present second modification, connecting portions 81 are provided in a matrix manner to hold corner portions of second acoustic matching layers 73 to be connected (four second acoustic matching layers 73 in the second modification), of a plurality of second acoustic matching layers 73 (piezoelectric elements 71) provided in a matrix manner, as illustrated in FIG. 21. According to the second modification, a disposition area of the connecting portion 81 is smaller than that of the connecting portion 79, and thus propagation influence of ultrasound waves by the connecting portion can be made small, and the ultrasound waves can be emitted or received in a wider range. Note that, in the second modification, the example in which the shape formed by an outer edge of the connecting portion 81 forms a rectangle (see FIG. 21) has been described. However, the shape formed by an outer edge of the connecting portion 81 may be a shape other than the rectangle, such as a circle, an oval, or a cross.

### (Third Modification of Third Embodiment)

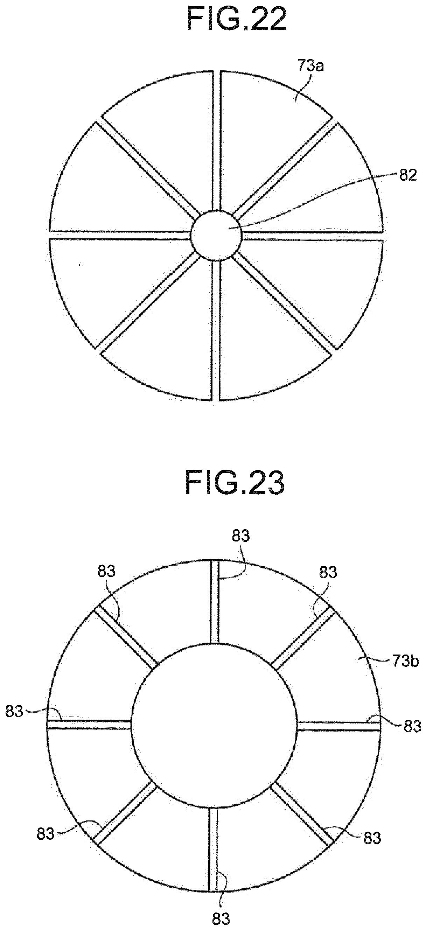

FIG. 22 is a top view schematically illustrating a principal portion of an ultrasound transducer according to a third modification of the third embodiment of the present invention. In the above-described third embodiment, the example in which the plurality of piezoelectric elements 71 is arranged in a matrix manner, and the shape formed by the outer edge (the shape formed by the outer edge in top view) is a substantially rectangle, and the ultrasound transducer having the first acoustic matching layer 72 and the second acoustic matching layer 73 are laminated in order on the principal plane of the piezoelectric elements 71 has been described. However, the shape formed by the outer edge of the plurality of piezoelectric elements 71 may be a circle or an oval. The third modification will be described using a case in which the shape formed by the outer edge of the plurality of piezoelectric elements 71 is a circle. In the third modification, second acoustic matching layers 73a laminated on the piezoelectric elements via first acoustic matching layers form a shape obtained by equally dividing a principal plane forming a circle into eight parts in a radial direction, and a connecting portion 82 that holds the plurality of second acoustic matching layers 73a is provided in a central portion of the circle. According to the third modification, the connecting portion 82 collectively holds the adjacent second acoustic matching layers 73a, thereby to maintain a pitch of the adjacent second acoustic matching layers 73a to a pitch at the time of division even if the layers are bent.

### (Fourth Modification of Third Embodiment)

FIG. 23 is a top view schematically illustrating a principal portion of an ultrasound transducer according to a fourth modification of the third embodiment of the present invention. In the third modification of the third embodiment, the example in which the shape formed by the outer edge of the plurality of piezoelectric elements 71 is a circle or an oval has been described. However, the shape formed by the outer edge of the plurality of piezoelectric elements 71 may be a hollow annular shape. In the fourth modification, second acoustic matching layers 73b laminated on piezoelectric elements via first acoustic matching layers form a shape obtained by equally dividing a principal plane with an outer edge forming a circle into eight parts in a radial direction, and a plurality of connecting portions 83 that holds the adjacent second acoustic matching layers 73b is provided. According to the fourth modification, the plurality of connecting portions 83 holds the adjacent second acoustic matching layers 73b, respectively, thereby to maintain a pitch of the adjacent second acoustic matching layers 73b to a pitch at the time of division even if the layers are bent.

### (Fifth Modification of Third Embodiment)

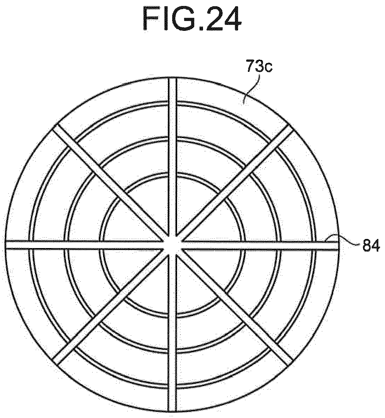

FIG. 24 is a top view schematically illustrating a principal portion of an ultrasound transducer according to a fifth modification of the third embodiment of the present invention. In the third modification of the third embodiment, the example in which the shape formed by the outer edge of the plurality of piezoelectric elements 71 is a circle or an oval, and the principal plane forming a circle is equally divided into eight parts in a radial direction has been described. However, the principal plane may be further divided along a circumferential direction. In the fifth modification, a second acoustic matching layer 73c laminated on a piezoelectric element via a first acoustic matching layer is divided in a circumferential direction and in a radial direction. Connecting portions 84 according to the fifth modification radially extend in a radial direction from a center of a circle formed by the outer edge of the plurality of second acoustic matching layers 73c and hold the adjacent second acoustic matching layers 73c. According to the fifth modification, the connecting portions 84 hold the adjacent second acoustic matching layers 73c, respectively, thereby to maintain a pitch of the adjacent second acoustic matching layers 73c to a pitch at the time of division even if the layers are bent.

Although the embodiments for carrying out the present invention have been described so far, the present invention should not be limited only by the above-described embodiments and modifications. The present invention is not limited to the above-described embodiments and modifications, but may include various embodiments within the scope not deviating from the technical idea described in the claims. Further, the configurations of the embodiments and the modifications may be combined as appropriate.

In the above-described first to third embodiments and modifications, the plurality of ultrasound elements has been formed by dividing the base material into a matrix manner, or in the radial direction and/or the circumferential direction. However, an embodiment is not limited thereto, and the plurality of piezoelectric elements may be arranged in a zigzag manner or arranged in a diagonal lattice manner.

Further, in the first to third embodiments and the modifications described above, the piezoelectric element has been described as an example of an element that emits an ultrasound wave and converts the ultrasound wave having entered from an outside into an echo signal. However, the piezoelectric element is merely an example, and an element manufactured in a manner of micro electro mechanical systems (MEMS), for example, capacitive micromachined ultrasonic transducers (C-MUT) may be employed.

Further, in the above-described first to third embodiments and modifications, the acoustic lens has been disposed in the recessed portion formed by the connecting portion and the second acoustic matching layer. However, an embodiment is not limited thereto, and the acoustic lens may further cover the surface of the connecting portion, or the acoustic lens may cover the ultrasound elements and the backing material, that is, the acoustic lens may form an outer peripheral surface of an ultrasound transducer.

Further, as an ultrasound probe, an ultrasound miniature probe having a small diameter without including an optical system may be applied. The ultrasound miniature probe is usually inserted into biliary tract, bile duct, pancreatic duct, trachea, bronchus, urethra, and ureter, and is used for observing surrounding organs (pancreas, lung, prostate, bladder, lymph node, and the like).

Further, as the ultrasound probe, an extracorporeal ultrasound probe that irradiates a body surface of a subject with ultrasound waves may be applied. The external ultrasound probe is usually used for observing abdominal organs (liver, gall bladder, and bladder), breast (especially mammary gland), and thyroid gland.

### Industrial Applicability

The ultrasound transducer and the ultrasound probe according to the present invention are effective to improve the directional characteristic of the ultrasound elements, and maintain the pitch of the ultrasound elements on the side emitting ultrasound waves when the ultrasound elements are bent along the array direction to the pitch at the time of division.

### Reference Signs List

- 1: ENDOSCOPE SYSTEM
- 2: ULTRASOUND ENDOSCOPE
- 3: ULTRASOUND OBSERVATION APPARATUS
- 4: ENDOSCOPE OBSERVATION APPARATUS
- 5: DISPLAY DEVICE
- 6: LIGHT SOURCE DEVICE
- 7, 7a, 7b: ULTRASOUND TRANSDUCER
- 21: INSERTION PORTION
- 22: OPERATING UNIT
- 23: UNIVERSAL CABLE
- 24: CONNECTOR
- 31: ULTRASOUND CABLE
- 41: VIDEO CABLE
- 61: OPTICAL FIBER CABLE
- 70, 70a: ULTRASOUND ELEMENT
- 71: PIEZOELECTRIC ELEMENT
- 72: FIRST ACOUSTIC MATCHING LAYER
- 73, 73a, 73b, 73c: SECOND ACOUSTIC MATCHING LAYER
- 74: ACOUSTIC LENS
- 75, 75a: BACKING MATERIAL
- 76, 77, 78, 79, 80, 81, 82, 83, 84: CONNECTING PORTION
- 211: RIGID MEMBER
- 212: BEND PORTION
- 213: FLEXIBLE TUBE PORTION
- 221: BENDING KNOB
- 222: OPERATING MEMBER
- 223: TREATMENT TOOL INSERTION PORT
- 241: FIRST CONNECTOR PORTION
- 242: SECOND CONNECTOR PORTION
- 243: THIRD CONNECTOR PORTION

## Claims

1. An ultrasound transducer comprising:
a plurality of ultrasound elements, each of the ultrasound elements including at least an element configured to emit an ultrasound wave according to an input of an electrical signal and convert the ultrasound wave entered from an outside into an echo signal, and one or a plurality of acoustic matching layers laminated on the element and configured to match acoustic impedance between the element and an observation target; and
a connecting portion protruding to an opposite side of the element with respect to a plane passing through a surface of the acoustic matching layer, and configured to connect the ultrasound elements adjacent to each other among the plurality of ultrasound elements.

2. The ultrasound transducer according to claim 1, wherein the plurality of ultrasound elements form a shape bent along an array direction.

3. The ultrasound transducer according to claim 1, wherein
the element is provided with a ground electrode for grounding, and
the connecting portion is formed using a conductive material, and is electrically connected with the ground electrode.

4. The ultrasound transducer according to claim 1, wherein the connecting portion is formed using a same material as the acoustic matching layer.

5. The ultrasound transducer according to claim 1, wherein
the ultrasound element has a plurality of acoustic matching layers, and
the connecting portion is formed using a same material as the acoustic matching layer to be connected.

6. The ultrasound transducer according to claim 1, further comprising:
a backing material configured to attenuate ultrasound vibration caused by an operation of the element, wherein
the connecting portion is formed using a same material as the backing material.

7. The ultrasound transducer according to claim 1, further comprising:
an acoustic lens configured to emit the ultrasound wave passed through the acoustic matching layer to an outside, wherein
the connecting portion is formed using a same material as the acoustic lens.

8. An ultrasound probe comprising the ultrasound transducer according to claim 1 at a distal end.

9. The ultrasound probe according to claim 8, further comprising an ultrasound endoscope including the ultrasound transducer at a distal end, and an insertion portion to be inserted into a subject.
